# EUROPEAN PATENT SPECIFICATION

(11) **EP 3 372 690 B1**
(45) Date of publication and mention of the grant of the patent: **12.03.2025**
(21) Application number: 16861627.4
(22) Date of filing: 04.11.2016
(51) Int. Cl.: C12Q 1/6809, C12Q 1/6811, C12Q 1/6827, G16B 20/00, G16B 30/00, C12Q 1/6883

(54) **METHOD OF DETECTING CHROMOSOME ABNORMALITY IN EMBRYO BY USING BLASTOCYST CULTURE**
VERFAHREN ZUR DETEKTION VON CHROMOSOMABNORMALITÄT IN EINEM EMBRYO DURCH VERWENDUNG EINER BLASTOZYSTENKULTUR
PROCÉDÉ DE DÉTECTION D'UNE ANOMALIE CHROMOSOMIQUE DANS UN EMBRYON À L'AIDE D'UNE CULTURE DE BLASTOCYSTES

(30) Priority: 05.11.2015 CN 201510746098
(43) Date of publication of application: 12.09.2018
(73) Proprietor: Xukang Medical Science & Technology (Suzhou) Co., Ltd., Suzhou, Jiangsu 215000 (CN)
(72) Inventor: LU, Sijia, Suzhou Jiangsu 215000 (CN); CAI, Liyi, Suzhou Jiangsu 215000 (CN); YAO, Bing, Suzhou Jiangsu 215000 (CN)
(74) Representative: Keenway Patentanwälte Neumann Heine Taruttis
(86) International application number: PCT/CN2016/104753
(87) International publication number: WO 2017/076359

(56) References cited:
- WO-A1-2013/116889
- WO-A1-2015/067796
- WO-A1-2015/114574
- CN-A- 105 368 936
- CN-A- 105 368 936
- QIAGEN: "QIAamp-MinElute-Virus-Spin-Handbook.pdf", 1 January 2014 (2014-01-01), pages 1 - 32, XP055565097, Retrieved from the Internet <URL:http://2014.igem.org/wiki/images/7/7b/NYM14_EN-QIAamp-MinElute-Virus-Spin-Handbook.pdf> [retrieved on 20190306]
- LUCA GALLUZZI ET AL: "Extracellular embryo genomic DNA and its potential for genotyping applications", FUTURE SCIENCE OA, vol. 1, no. 4, 1 November 2015 (2015-11-01), XP055564243, DOI: 10.4155/fso.15.62
- HAITAO WU ET AL: "Medium-Based Noninvasive Preimplantation Genetic Diagnosis for Human [alpha]-Thalassemias-SEA :", MEDICINE., vol. 94, no. 12, 1 March 2015 (2015-03-01), US, pages e669, XP055564337, ISSN: 0025-7974, DOI: 10.1097/MD.0000000000000669
- JUANJUAN XU ET AL: "Noninvasive chromosome screening of human embryos by genome sequencing of embryo culture medium for in vitro fertilization", PROCEEDINGS OF THE NATIONAL ACADEMY OF SCIENCES OF THE UNITED STATES OF AMERICA, vol. 113, no. 42, 29 September 2016 (2016-09-29), US, pages 11907 - 11912, XP055380709, ISSN: 0027-8424, DOI: 10.1073/pnas.1613294113
- XU, J. ET AL.: "Noninvasive Chromosome Screening of Human Embryosby Genome Sequencing of Embryo Culture Medium for in Vitro Fertilization", PROC NATL ACAD SCI USA, vol. 113, no. 42, 18 October 2016 (2016-10-18), pages 11907 - 11912, XP055380709

## Description

### Technical field

The present invention relates to the field of biomedicine and molecular cell biology, and in particular relates to a method for detecting and analyzing the state of an embryo chromosome by using a blastocyst culture solution.

### Background

The IVF technique is a powerful technique against infertility. The technical process is shown as follows: firstly, obtaining multiple eggs (usually 8 to 15) from the mother and then fertilizing the eggs with the father's sperm *in vitro.* When the fertilized eggs are grown in vitro culture solution for 5 days, the embryo is a cystic structure (i.e., blastocyst) consisting of about 80 to 100 cells. After 2-3 blastocysts are implanted into the mother's uterus, ideally, one to three of the blastocysts placed into the uterus can be successfully developed during normal pregnancy until birth. However, due to various reasons, the success rate for the implantation of the blastocyst into the uterus and the birth of the fetus is not high, usually only about 40%. In addition to the mother's own health reasons, the quality of the fertilized egg is one of the important reasons leading to the failure of blastocyst development.

The chromosome of the fertilized egg is derived from the maternal egg and the patrilineal sperm, and chromosomal abnormalities from any one will lead to chromosomal abnormalities of fertilized eggs. There are 44 autosomes, that is, 22 pairs of autosomes (called diploids) and two sex chromosomes (XY for male, XX for female) in fertilized eggs of the normal, each embryonic cell and each cell of fetus, infants up to adults. In an abnormal situation, more than or less than a diploid may occur in all or part of any chromosome, which is called aneuploidy abnormality. Aneuploidy abnormality is the most common form of chromosome abnormality that leads to failure of embryonic development. In the conventional IVF technique, depending only on the morphological observations under microscope, 2-3 relatively normal embryos are selected from multiple (usually 8-15) embryos and implanted into the mother's uterus. The normal morphology under the microscope can not reflect whether the chromosomes are normal. Incorrect selection of embryos with normal morphology but chromosomal abnormalities into the mother's uterus has caused many test-tube babies to fail to conceive.

In recent years, a number of techniques have been established, collectively referred to as a Preimplantation Genetic Screen (PGS), for the detection of the chromosomal status of cultured embryos *in vitro,* thereby implanting the screened embryos with normal chromosomes into the mother's uterus to improve success rate of the conception. Studies have shown that the operation of test-tube baby that is implanted into the uterus through PGS can increase the success rate to more than 60%. Various PGS methods comprise immunofluorescence (FISH), chip detection, and second-generation sequencing and the like. The biological samples necessary for the above-mentioned various detections are one to several cells collected from embryos cultured *in vitro,* and the detection for this small number of cells reflects whether the chromosome of the entire embryo is normal.

Specifically, embryonic trophoblast cells (trophoblast) can be extracted when the fertilized eggs that have been cultured for 5 days *in vitro* have been developed to the blastocyst stage. The general operation is to use a capillary glass tube to lyse cells from one to several trophoblast cells under a microscope to release trace amounts of DNA. After the trace amounts of DNA are subjected to a whole genome amplification, the chromosomal status of the cell can be detected using nucleic acid chips or second-generation sequencing (see patent application of CN104711362A, published on June 17, 2015). In theory, the chromosomal state in several cells that are sucked out is consistent with other cells in the embryo. Whether the chromosome status of the embryo is normal can be known through the detection for these cells. It is generally believed that the development of an embryo won't be adversely affected by taking several trophoblast cells at this time. From the health status of the born babies, this operation does not have a health effect. However, the occurrence time for this technology is still short (only a few years). Whether there is a long-term impact on people's lifelong health is still to be observed.

In addition, a method for detecting embryo quality using blastochyle has been developed, that is, firstly, obtaining a free DNA in blastochyle, i.e., using a micro-puncture technique under a micromanipulator and using a sterile needle to obtain a free DNA in blastochyle (see the invention patent application of CN104450923A, published on March 25, 2015; and journal articles of Luca Gianaroli, M. Cristina Magli, Alessandra Pomante, et al. Blastocentesis: a source of DNA for preimplantation genetic testing Results from a pilot study. Fertility and Sterility, 2014, 102(6):1692-1698.). However, the blastochyle is a liquid in the blastocyst cavity. It is still necessary to make a hole or puncture on the blastocyst to obtain the blastochyle, and its interventional will still cause inevitable damage to the embryo.

In summary, the main disadvantages of the prior art are:
1. The technical requirements for the operation of embryos during cell sampling are relatively high. The erroneous operation and rough operation can lead to serious damage to embryos, and excessive damage can cause the termination of embryonic development.
2. Even with good operation, cell sampling inevitably causes cell loss and minor damage to the embryo. Although there is no evidence that cell loss and minor damage may have adverse effects on embryonic development and postnatal health, the occurrence time for this technology is short (only a few years), and whether there will be a long-term effect on people's lifetime health is still to be observed.
3. In rare cases, there are cases where the chromosomal status of the sampled several cells is different from that of other cells in the embryo, resulting in erroneous detection results.

Therefore, a non-invasive technical means that does not damage the embryo itself and can check the chromosome status of the embryo is a practical need to eliminate the hidden dangers of health and ensure the safety of embryo detection.

WO2015114574 A teaches preimplantation assessment of embryos through detection of free embryonic DNA which requires the extraction and separation of nucleic acid from the spent blastocyst culture fluid prior to whole genome amplification.

### Summary of invention

The object of the present invention is to provide a method for detecting chromosomal abnormalities in embryos using blastocyst culture liquid, which will not do any damage to the embryos, has a simple operation, and has higher safety and reliability.

To achieve the above object, the present invention provides a method for detecting chromosomal abnormality of an embryo using blastocyst culture fluid, which comprises the following steps:
(1) Obtaining a blastocyst culture fluid: fertilized eggs are obtained by a single sperm injection method, cultured to the blastomere stage on day 3, and then transferred to a newly prepared blastocyst culture microdroplet for blastocyst culture. At this time, on the third day, it is necessary to change the solution to remove the contamination of the detached granular cells and unfertilized sperm;
   The embryos that form the blastocysts are taken and transferred to a new blastocyst culture solution or into a vitrified cryopreservation process. The remaining original blastocyst culture fluid is approximately 1 microliter to 500 microliters, preferably 10 microliters to 200 microliters, i.e., which is a sample to be collected for preimplantation genetic screening (PGS);
(2) Collection of blastocyst culture fluid: The original blastocyst culture fluid obtained in step (1) is transferred to a lysis solution, and after centrifugation, the sample is subjected to the next step of whole genome amplification;
(3) whole genome amplification of trace DNAs in blastocyst culture fluid: lyase is added to a mixture of blastocyst culture fluid obtained in step (2) and a lysis solution, mixed and incubated, then lyase is inactivated. The lysate is removed and added to a PCR reaction tube for PCR reaction; and
(4) analyzing DNA products obtained from whole genome amplification to determine whether the chromosome status of the embryo is normal: second-generation sequencing, nucleic acid chip or immunofluorescence detection is used for analysis.

The embryos that form the blastocysts are taken after 2-3 days of solution exchange, and transferred to a new blastocyst culture solution or into a vitrified cryopreservation process, and the remaining original blastocyst culture fluid is approximately 1 microliter to 500 microliters, preferably 10 µ l to 200 µ l, i.e., which is a sample to be collected for preimplantation genetic screening (PGS);
wherein, the components of the lysis solution in step (2) are 25-45 mM of Tris-Cl, pH 7.0-8.0, 0.5-3 mM of EDTA, 10-25 mM of KCl and a detergent with a concentration of 0.05%-5%, the detergent is one or more selected from a group consisting of Triton X-100, Triton X-114, Tween 20, NP40, and SDS. Preferably, the components of the lysis buffer are 40 mM of Tris-Cl, pH 7.2, 1 mM of EDTA, 15 mM of KCl, and 3% of Triton X-100.

In a preferred embodiment, in step (3), the primers used comprise NG primers, NT primers, and amplification primers,
wherein the NG primers and the NT primers comprise a universal sequence and a variable sequence from 5' end to 3' end, wherein the universal sequence consists of three or two of the four bases of G, A, C, and T; provided that the universal sequence does not comprise G and C at the same time;

The variable sequence of the NG primers is selected from a group consisting of: (N)nGGG, (N)xGTGG(N)y, or a combination thereof; while the variable sequence of the NT primers is selected from a group consisting of: (N)nTTT, (N) mTNTNG, or a combination thereof; wherein N is any nucleotide that can be base-paired with a natural nucleic acid, each n is independently a positive integer selected from 3-17, each m is independently a positive integer selected from 3-15, and each of x and y is a positive integer selected from 3-13, respectively;

Whereas, the amplification primer contains the universal sequence instead of the variable sequence.

The lyase in step (3) is one or more selected from a group consisting of Proteinase K, Qiagen Protease, pepsin, papain, trypsin and lysozyme, the concentration of the lyase is 1-25 µg/ml, preferably 20 µg/ml; the incubation temperature in step (3) is 30-60°C, the incubation time is 1min-12h, the inactivation temperature is 75-95°C, and the inactivation time is 1-15 min; preferably the incubation temperature is 40°C, the incubation time is 3h, the inactivation temperature is 90°C, and the inactivation time is 5 min.
when the PCR reaction is performed in step (3), the PCR reaction tube comprises an amplification mixture, 0.5%-20% of a PCR inhibitor antagonist, 5-20 mM of dNTP, 5-100 µM of NG and NT primers, 50-200 µM of amplification primers, 0.5-10 units of nucleic acid polymerase, and the PCR inhibitor antagonist is one or more selected from a group consisting of DMSO, betaine, formamide, glycerol and albumin, the nucleic acid polymerase is one or more selected from a group consisting of Phi29 DNA polymerase, Bst DNA polymerase, Vent polymerase, Deep Vent polymerase, Klenow Fragment DNA polymerase I, MMLV reverse transcriptase, AMV reverse transcriptase, HIV reverse transcriptase, Phusion^{®} super-fidelity DNA polymerase, Taq polymerase, E.coli DNA polymerase, LongAmp Taq DNA polymerase, and OneTaq DNA polymerase.

The components of the amplification mixture are 10-25 mM of Tris-HCl, 5-25 mM of (NH₄)₂SO₄, 5-30 mM of KCl, 0.5-5 mM of MgSO₄, 0.1 %-20% of DMSO and 0.05-5% of Triton X-100. Preferably, the components of the amplification mixture are 15 mM of Tris-HCl, 15 mM of (NH₄)₂SO₄, 20 mM of KCl, 1 mM of MgSO₄, 5% of DMSO and 2% of Triton X-100.

The NG and NT primer comprise a universal sequence and a variable sequence from 5' end to 3' end, wherein the universal sequence consists of 3 or 2 of the 4 bases of G, A, C and T, provided that the universal sequence does not simultaneously comprise G and C; the amplification primer contains the universal sequence without the variable sequence. The variable sequence is selected from a group consisting of: (N)nGGG, (N)nTTT, (N)mTNTNG, (N)xGTGG(N)y, wherein N is any nucleotide that can be base-paired with a natural nucleic acid, n is a positive integer selected from 3-17, m is a positive integer selected from 3-15, each of x and y is a positive integer selected from 3-13, respectively.

Preferably, the NG and NT primer comprise the sequence of SEQ ID NO: 1 [GTGAGTGATGGTTGAGGTAGTGTGGAGNNNNNNNN], SEQ ID NO: 2 [GTGAGTGATGGTTGAGGTAGTGTGGAGNNNNNGGG], SEQ ID NO: 3 [GTGAGTGATGGTTGAGGTAGTGTGGAGNNNNNTTT], SEQ ID NO: 4 [GTGAGTGATGGTTGAGGTAGTGTGGAGNNNTNTNG], or SEQ ID NO: 5 [GTGAGTGATGGTTGAGGTAGTGTGGAGNNNGTGGNN], wherein N is any nucleotide that can be base-paired with a natural nucleic acid; and the amplification primer has the sequence of SEQ ID NO: 6 [GTGAGTGATGGTTGAGGTAGTGTGGAG] from 5' to 3'.

The thermocycling procedure of whole genome amplification in step (3) is shown as follows:
(1) reacting at a first denaturation temperature between 90-98°C for 5-20 seconds;
(2) reacting at a first annealing temperature of 5-15°C for 5-60s, reacting at a second annealing temperature of 15-25°C for 5-60s, reacting at a third annealing temperature of 25-35°C for 30-80s, reacting at a fourth annealing temperature of 35-45 °C for 5-60s, and reacting at a fifth annealing temperature of 45-55 °C for 5-60s;
(3) reacting at a first extension temperature of 55-80°C for 10-150 min;
(4) reacting at a second denaturation temperature of 90-98°C for 5-30 s;
(5) reacting at a sixth annealing temperature of 45-70°C for 10-30 s;
(6) reacting at a second extension temperature of 60-80°C for 1-10 minutes;
(7) repeating steps (4) to (6) for 5 to 50 cycles;
(8) continuing the extension reaction at a temperature of 60-80 °C for 1-10min; and
(9) refrigerating and storing the amplified product at 0-5°C.

Preferably the thermocycling procedure of whole genome amplification in step (3) is shown as follows:
(1) reacting at a first denaturation temperature between 95 °C for 10 seconds;
(2) reacting at a first annealing temperature of 10 °C for 45s, reacting at a second annealing temperature of 20 °C for 45s, reacting at a third annealing temperature of 30°C for 60s, reacting at a fourth annealing temperature of 40 °C for 45s, and reacting at a fifth annealing temperature of 50 °C for 45s;
(3) reacting at a first extension temperature of 62 °C for 90 min;
(4) reacting at a second denaturation temperature of 95 °C for 20 s;
(5) reacting at a sixth annealing temperature of 59 °C for 20 s;
(6) reacting at a second extension temperature of 72 °C for 3 min;
(7) repeating steps (4) to (6) for 10 to 30 cycles;
(8) continuing the extension reaction at a temperature of 72 °C for 5 min; and
(9) refrigerating and storing the amplified product at 4 °C.

The amplification product obtained from the above step (9) is subjected to steps such as routine database construction, sequencing, and data analysis for the detection of copies of each chromosome and local chromosomes in the sample genome according to the technical requirements including but not limited to Illumina Hiseq, Miseq, Life Technology PGM, Proton sequencer. Copy number of the normal chromosomes and local chromosomes is 2. When the copy number is greater than 2 (such as ≥ 2.5) or less than 2 (such as ≤ 1.8), it is an abnormal copy number, that is, abnormal chromosomes. This normal or abnormal detection result represents the normal or abnormal chromosome of the culture fluid-derived embryo. If embryos of chromosome abnormalities are implanted in the matrix, embryo implantation failure, miscarriage, and other adverse consequences can occur. Only embryos with normal chromosome are implanted in the matrix, there will be a higher chance of successful conception.

In the present invention, embryo-derived free DNA from early embryonic *in vitro* culture fluid (blastocyst fluid) at the early stage of embryo is detected to determine the chromosome condition of the embryo (the presence of whole or partial chromosome aneuploidy). Since embryos release a very small amount (about several tens of picograms) of DNA into the blastocyst culture fluid during early development of the embryo *in vitro* culture, in order to use such a small amount of DNA for the detection of chromosome aneuploidy, the DNA must be uniform amplified first at a large scale. However, the volume of the blastocyst culture fluid is about 30 microliters, so that the embryo-derived DNA in the culture fluid is highly diluted. At the same time, the components of the embryonic culture fluid are complex, and some of the components will inhibit DNA amplification. The technical solution of the present invention overcomes the above-mentioned technical problems and successfully establishes a technical method for detecting embryonic chromosome aneuploidy from the blastocyst culture fluid.

Therefore, compared with the prior art, the present invention avoids the cell loss and damage to the embryo caused by the conventional PGS detection and sampling method, and simplifies the operation of the PGS sample acquisition; in addition, since the blastocyst culture fluid is originally a waste during the *in vitro* embryo culture stage of IVF operation, this waste is detected by the technology of the present invention, thereby hardly adding extra trouble to the clinic and making the evaluation of the chromosome status of the corresponding embryo possible.

### Description of Figure

The present invention will be further described in detail with reference to the accompanying drawings and specific embodiments.

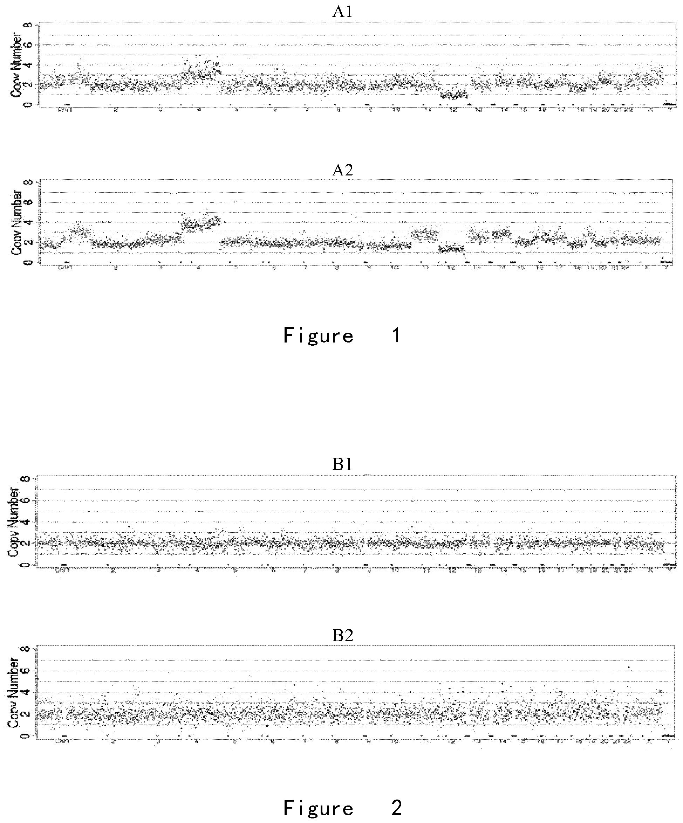

Figure 1 shows an analysis of the results for chromosome detection of sample A using blastocyst culture fluid and blastocyst cells, respectively, in Example 1 of the present invention;
Figure 2 shows an analysis of the results for chromosome detection of sample B using blastocyst culture fluid and blastocyst cells, respectively, in Example 1 of the present invention.

### Detailed Description

After extensive and in-depth researches, through a large number of screenings and tests, the present inventors have unexpectedly found that embryos are cultured in a small amount of culture fluid, and a very small amount of the culture fluid is taken out for detection, and it is found that the detection results for the obtained chromosomal abnormality have extremely high accuracy. Based on this, the present inventors completed the present invention.

### Terms

The blastocyst culture fluid used in the detection technique of the present invention is a cell-free blastocyst culture fluid.

### Detection method

The present invention provides a method for the gene detection of a depleted medium (i.e., a culture fluid separated from the culture system), thereby identifying whether the chromosome of the embryo is abnormal, wherein the depleted medium is the medium after the blastocyst is cultured.

In the present invention, the gene detection method for the " depleted " culture fluid (i.e., a culture fluid separated from the culture system), which is the culture after the blastocyst is cultured, is not particularly limited, and can be detected by a conventional method, such as a second-generation sequencing, a nucleic acid chip, an immunofluorescence detection, a fluorescence PCR detection, a first-generation sequencing, a third-generation sequencing, a mass spectrometry detection, or a combination thereof.

In one embodiment, the detection method comprises the following steps:
(1) Obtaining a blastocyst culture fluid: a fertilized egg is obtained by a single sperm injection method, and cultured to the blastomere stage on day 3, and then transferred to a newly prepared blastocyst culture microdroplet for blastocyst culture. At this time, on the third day, it is necessary to change the solution to remove the contamination of the detached granular cells and unfertilized sperm;
   The embryos that form the blastocysts are taken and transferred to a new blastocyst culture solution or into a vitrified cryopreservation process. The remaining original blastocyst culture fluid is approximately 1 microliter to 500 microliters, preferably 10 microliters to 200 microliters, i.e., which is a sample to be collected for preimplantation genetic screening (PGS);
(2) Collecting a blastocyst culture fluid: transferring the original blastocyst culture fluid obtained in step (1) to a lysis solution, and after centrifugation, the sample is subjected to the next step of whole genome amplification;
(3) whole genome amplification of trace DNAs in blastocyst culture fluid: lyase is added to a mixture of the blastocyst culture fluid obtained in step (2) and a lysis solution, mixed and incubated, then lyase is inactivated, and the lysate is removed and added to a PCR reaction tube for PCR reaction; and
(4) analyzing DNA products obtained from whole genome amplification to determine whether the chromosome status of the embryo is normal: second-generation sequencing, nucleic acid chip or immunofluorescence detection is used for analysis.

In a preferred embodiment, the embryos that form the blastocysts are removed after 2-3 days of solution exchange, and transferred to a new blastocyst culture solution or into a vitrified cryopreservation process, and the remaining original blastocyst culture fluid is approximately 1 microliter to 500 microliters, preferably 10 µl to 200 µl, which is the sample that needs to be collected for Preimplantation Genetic Screening (PGS).

### The major advantages of the present invention include:

(1) In the present invention, the embryos are cultured in a very small amount of the culture solution, and an extremely small amount of the culture fluid is detected, and the detection result of chromosome abnormality has unexpectedly an extremely high accuracy.
(2) A single embryo culture system is used in the present invention, i.e., only one embryo is cultured in one droplet of the culture fluid, and the detection result of the chromosome abnormality obtained by this system is more accurate.

The invention is further illustrated by the following examples. These examples are only intended to illustrate the invention, but not to limit the scope of the invention. For the experimental methods in the following examples the specific conditions of which are not specifically indicated, they are performed under routine conditions, e.g., those described by Sambrook. et al., in Molecular Cloning: A Laboratory Manual, New York: Cold Spring Harbor Laboratory Press, 1989, or as instructed by the manufacturers. Unless otherwise indicated, percentages and parts are percentages by weight and parts by weight.

Unless otherwise specified, the materials and reagents used in the examples of the present invention are all commercially available products.

### Example 1

Two *in vitro* fertilized embryos samples, A and B were selected, and chromosomal status thereof was assessed by the methods of blastocyst cell detection and blastocyst culture fluid detection, respectively, the specific steps were shown as follows:
1. Obtaining a blastocyst culture fluid
1) a fertilized egg obtained by a single sperm injection method was cultured to the blastomere stage on day 3, and the embryo was transferred to a newly prepared blastocyst culture microdroplet for blastocyst culture.
2) The embryos that form the blastocysts were removed and transferred to a new blastocyst culture solution or into a vitrification cryopreservation process. The remaining original blastocyst culture fluid (about 30ul) was sample A and B that need to be collected for PGS. Preferably, after 2-3 days of fluid exchange, the blastocysts-forming embryos were removed.

2. Collecting blastocyst culture fluid
1) the collection tube containing 10 µl of lysis solution (40 mM of Tris-Cl, pH 7.2, 1 mM of EDTA, 15 mM of KCl, and 3% of Triton X-100) was placed for 2 min at room temperature. After the lysis solution is thawed, the sample collection tube was placed in a mini-centrifuge and centrifuged for 30 seconds to ensure that all of the lysis solution was at the bottom of the tube.
2) all of the original blastocyst culture fluid from 2) of step 1 was transferred to the lysis solution using a mouth pipette.
3) the name of the sample was marked on the collection tube with a marker pen, centrifuged for 30s using the microcentrifuge, and the sample can immediately be subjected into the next step of whole genome amplification or be stored frozen at -20°C or -80°C.

3. Whole genome amplification of trace DNAs in blastocyst culture fluid
1) A mixture of the blastocyst culture fluid and the lysis solution was thawed at room temperature.
2) Protease was added into the tube and mixed up and down.
3) The tube was incubated for 3 h at 40 °C.
4) The lyase was inactivated by placing the tube at 90°C for 5 min.
5) The lysate was removed from the tube and added to a PCR reaction tube.
6) An amplification mixture (15 mM of Tris-HCl, 15 mM of (NH₄)₂SO₄, 20 mM of KCl, 1 mM of MgSO₄, 5% of DMSO and 2% of Triton X-100), 5% of DMSO, 10 mM of dNTP, 50 µM of NG (5' -GT GAG TGA TGG TTG AGG TAG TGT GGA GNNNNNGGG-3' and NT (5' -GT GAG TGA TGG TTG AGG TAG TGT GGA GNNNNNTTT-3' ) primers, 100 µM of amplification primers (5 '-GT GAG TGA TGG TTG AGG TAG TGT GGA G-3'), 1 unit of Bst DNA polymerase, 1 unit of Deep VentR were added to the PCR reaction tube.
7) The PCR reaction tube was placed in the PCR instrument for whole-genome amplification. The thermal cycle program was as follows:

| | | |
|---|---|---|
| 95°C - | 10 seconds | |
| 10°C - | 45 seconds | |
| 20°C - | 45 seconds | |
| 30°C - | 60 seconds | |
| 40°C - | 45 seconds | |
| 50°C - | 45 seconds | |
| 62°C - | 90 minutes | 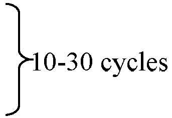 |
| 95°C - | 20 seconds | |
| 59°C - | 20 seconds | |
| 72°C - | 3 minutes | |
| 72°C - | 5 minutes | |
| 4°C | ∞ | |

4. The amplified DNA product was subjected to a second-generation sequencing according to conventional methods to identify whether the chromosome status of the embryo is normal.

The results of the second-generation sequencing data showed that in sample A, abnormalities in multiple chromosome can be detected by both of the blastocyst culture fluid detection method (Figure A1) and the blastocyst cell detection method (Figure A2); however, in sample B, chromosomes were judged to be normal by the blastocyst culture fluid detection method (Figure B1) and the blastocyst cell detection method (Figure B2). The above results showed that identical results for the identification of embryonic chromosome status were obtained using the blastocyst culture fluid detection and blastocyst cell detection methods, thereby further confirming that the non-invasive detection method was accurate and reliable.

### Example 2

Forty-two *in vitro* cultured embryos were randomly selected and compared between the methods of the blastocyst culture fluid detection and the blastocyst cell detection according to Example 1, respectively. Logically, correspondences between the detection results can be presented in four combinations;
the first type, both of cell detection and detecton results of the culture fluid showed abnormal.

The second type, both of cell detection and detection results of the culture fluid showed normal.

The third type, cell detection showed normal and detection results of the culture fluid showed abnormal.

The fourth type, cell detection showed abnormal, and detection results of the culture fluid showed normal.

In 42 comparison results, the distribution of the number of cases of these four correspondences was shown in Table 1:

**Table 1**

| result correspondence | number of cases | percentage |
|---|---|---|
| the first type | 15 | 35.7% |
| the second type | 21 | 50.0% |
| the third type | 4 | 9.5% |
| the fourth type | 2 | 4.8% |
| in total | 42 | 100% |

The results showed that when the cell assay was used as the gold standard, the sensitivity of the culture fluid detection was calculated as 88.2%, the specificity was 84.0%, the positive predictive value was 78.9, and the negative predictive value was 91.3%. Although none of the indicators is 100%, the non-invasive method provides detection results sufficiently close to the gold standard, which is sufficient to confirm the beneficial value of the present invention.

### Example 3

Embryos of 8 patients suffering from fertility difficulties due to different reasons were subjected to embryo culture fluid detection in accordance with the method of Example 1 in the present invention, and embryos with normal chromosome were selected and implanted into the mother's uterus based on the detection results.

The results were shown in Table 2.

**Table 2**

| NO.: | Indications | Number of embryo transfer | Number of transpla nted embryos | impla ntatio n | clinic al pregn ancy | sustai ned pregn ancy | live birth |
|---|---|---|---|---|---|---|---|
| 1 | male chromosome balanced translocation, t(14: 15) | 1 | 1 | 1 | Yes | Yes | Yes |
| 2 | male azoospermia, 46,XY, 15p+ | 1 | 1 | 1 | Yes | Yes | Yes |
| 3 | male chromosome balanced translocation, t(20;22) | 0 | 0 | 0 | No | No | No |
| 4 | male chromosome inversion, inv(p12q13) | 1 | 1 | 1 | Yes | Yes | Yes |
| 5 | female chromosome balanced translocation, t(1;18) | 2 | 2 | 0 | No | No | No |
| 6 | recurrent abortion (three miscarriages) | 1 | 1 | 1 | Yes | Yes | Yes |
| 7 | male 47, XYY | 2 | 2 | 1 | Yes | Yes | Yes |
| 8 | male 46, XY, ins(6;7) | 0 | 0 | 0 | No | No | No |

In general, 80% of the gametes (i.e., sperms or eggs) in patients with balanced translocations have chromosome aneuploidy, and the success rate of natural conception is low. The conventional IVF method also fails to identify embryos with chromosome aneuploidy, and the success rate is very low.

The results of the present invention showed that patients of NO.3 and 8 did not undergo embryo transfer because no high-quality fertilized eggs were detected and the chromosomes of the embryos were abnormal. In addition, the results of the remaining 6 patients fully demonstrated that a patient can successfully conceive after an embryo with normal chromosome selected by the method of the present invention was implanted into the patient for only one time and the success rate of embryo transfer and the survival was 5/6 (i.e., 83.3%), that is, only one patient did not succeed.

Therefore, the results showed that a very high conception rate and embryo survival rate can be obtained through the method of the present invention.

The above description of the disclosed embodiments of the present invention enables those skilled in the art to implement or use the present invention. At the same time, the above are merely preferred embodiments of the present invention and are not intended to limit the embodiments of the present invention.

## Claims

1. A method for detecting the chromosomal abnormality in an embryo using blastocyst culture fluid, comprising the steps of:
(1) obtaining a blastocyst culture fluid: culturing a fertilized egg to the blastomere stage on day 3, and then transferred to a newly prepared blastocyst culture microdroplet for blastocyst culture, the embryo that forms the blastocyst is removed and transferred to a new blastocyst culture solution or into a vitrified cryopreservation process, and the remaining original blastocyst culture fluid is a sample to be collected for detection;
(2) collecting a blastocyst culture fluid: transferring the original blastocyst culture fluid obtained in step (1) to a lysis solution, and after centrifugation, the sample is subjected to the next step of whole genome amplification;
(3) whole genome amplification of trace DNAs in blastocyst culture fluid: lyase is added to a mixture of the blastocyst culture fluid obtained in step (2) and a lysis solution, mixed and incubated, then lyase is inactivated, and the lysate is removed and added to a PCR reaction tube for genome amplification reaction;
(4) analyzing DNA products obtained from whole genome amplification to determine whether the chromosome status of the embryo is normal: second-generation sequencing, nucleic acid chip or immunofluorescence detection is used for analysis.

2. The method of claim 1, wherein the components of the lysis solution in step (2) are 25-45 mM of Tris-Cl with a pH of 7.0-8.0, 0.5-3 mM of EDTA, 10-25 mM of KCl and a detergent with a concentration of 0.05%-5%, and the detergent is one or more selected from a group consisting of Triton X-100, Triton X-114, Tween 20, NP40, and SDS.

3. The method of claim 2, wherein the components of the lysis solution are preferably 40 mM of Tris-Cl, pH 7.2, 1 mM of EDTA, 15 mM of KCl, and 3% of Triton X-100.

4. The method of claim 1, wherein the lyase in step (3) is one or more selected from a group consisting of Proteinase K, Qiagen Protease, pepsin, papain, trypsin and lysozyme, and the concentration of the lyase is 1-25µg/ml.

5. The method of claim 4, wherein the concentration of the lyase is preferably 20µg/ml.

6. The method of claim 1, wherein the incubation temperature in step (3) is 30-60°C, the incubation time is 1 min to 12 hrs, the inactivation temperature is 75-95°C, and the inactivation time is 1-15 mins.

7. The method of claim 6, wherein preferably, in step (3), the incubation temperature is 40°C, the incubation time is 3hrs, the inactivation temperature is 90°C, and the inactivation time is 5 mins.

8. The method of claim 1, wherein, when the PCR reaction is performed in step (3), the PCR reaction tube comprises an amplification mixture, 0.5%-20% of a PCR inhibitor antagonist, 5-20 mM of dNTP, 5-100 µM of NG and NT primers, 50-200 µM of amplification primers, 0.5-10 units of nucleic acid polymerase, and the PCR inhibitor antagonist is one or more selected from a group consisting of DMSO, betaine, formamide, glycerol and albumin, the nucleic acid polymerase is one or more selected from a group consisting of Phi29 DNA polymerase, Bst DNA polymerase, Vent polymerase, Deep Vent polymerase, Klenow Fragment DNA polymerase I, MMLV reverse transcriptase, AMV reverse transcriptase, HIV reverse transcriptase, Phusion^{®} super-fidelity DNA polymerase, Taq polymerase, E.coli DNA polymerase, LongAmp Taq DNA polymerase, and OneTaq DNA polymerase.

9. The method of claim 8, wherein the components of the amplification mixture are 10-25 mM of Tris-HCl, 5-25 mM of (NH4)2SO4, 5-30 mM of KCl, 0.5-5 mM of MgSO4, 0.1 %-20% of DMSO and 0.05-5% of Triton X-100.

10. The method of claim 9, wherein the components of the amplification mixture are preferably 15 mM of Tris-HCl, 15 mM of (NH4)2SO4, 20 mM of KCl, 1 mM of MgSO4, 5% of DMSO and 2% of Triton X-100.

11. The method of claim 8, wherein the NG and NT primers comprise a universal sequence and a variable sequence from 5' end to 3' end, and wherein the universal sequence consists of 3 or 2 of the 4 bases of G, A, C and T, provided that the universal sequence does not simultaneously comprise G and C; and the amplification primer comprises the universal sequence while not comprises the variable sequence.

12. The method of claim 11, wherein the variable sequence is selected from a group consisting of: (N)nGGG, (N)nTTT, (N)mTNTNG, (N)xGTGG(N)y, wherein N is any nucleotide that can be base-paired with a natural nucleic acid, n is a positive integer selected from 3-17, m is a positive integer selected from 3-15, and each of x and y is a positive integer selected from 3-13, respectively.

13. The method of claim 12, wherein the NG and NT primers comprise the sequence of SEQ ID NO: 1 [GTGAGTGATGGTTGAGGTAGTGTGGAGNNNNNNNN], SEQ ID NO: 2 [GTGAGTGATGGTTGAGGTAGTGTGGAGNNNNNGGG], SEQ ID NO: 3 [GTGAGTGATGGTTGAGGTAGTGTGGAGNNNNNTTT], SEQ ID NO: 4 [GTGAGTGATGGTTGAGGTAGTGTGGAGNNNTNTNG], or SEQ ID NO: 5 [GTGAGTGATGGTTGAGGTAGTGTGGAGNNNGTGGNN], wherein N is any nucleotide that can be base-paired with a natural nucleic acid; and the amplification primer has the sequence of SEQ ID NO: 6 [GTGAGTGATGGTTGAGGTAGTGTGGAG] from 5' to 3'.

14. The method of claim 1, wherein the thermocycling procedure of whole genome amplification in step (3) is shown as follows:
(1) reacting at a first denaturation temperature between 90-98°C for 5-20 seconds;
(2) reacting at a first annealing temperature of 5-15°C for 5-60s, reacting at a second annealing temperature of 15-25°C for 5-60s, reacting at a third annealing temperature of 25-35°C for 30-80s, reacting at a fourth annealing temperature of 35-45°C for 5-60s, and reacting at a fifth annealing temperature of 45-55 °C for 5-60s;
(3) reacting at a first extension temperature of 55-80°C for 10-150 min;
(4) reacting at a second denaturation temperature of 90-98°C for 5-30 s;
(5) reacting at a sixth annealing temperature of 45-70°C for 10-30 s;
(6) reacting at a second extension temperature of 60-80°C for 1-10 minutes;
(7) repeating steps (4) to (6) for 5 to 50 cycles;
(8) continuing the extension reaction at a temperature of 60-80°C for 1-10min; and
(9) refrigerating and storing the amplified product at 0-5°C.

15. The method of claim 14, wherein the thermocycling procedure of whole genome amplification in step (3) is shown as follows:
(1) reacting at a first denaturation temperature between 95 °C for 10 seconds;
(2) reacting at a first annealing temperature of 10 °C for 45s, reacting at a second annealing temperature of 20 °C for 45s, reacting at a third annealing temperature of 30°C for 60s, reacting at a fourth annealing temperature of 40 °C for 45s, and reacting at a fifth annealing temperature of 50 °C for 45s;
(3) reacting at a first extension temperature of 62 °C for 90 min;
(4) reacting at a second denaturation temperature of 95 °C for 20 s;
(5) reacting at a sixth annealing temperature of 59 °C for 20 s;
(6) reacting at a second extension temperature of 72 °C for 3 min;
(7) repeating steps (4) to (6) for 10 to 30 cycles;
(8) continuing the extension reaction at a temperature of 72 °C for 5 min; and
(9) refrigerating and storing the amplified product at 4 °C.

16. The method of claim 1. wherein, in step (3), the primers used in PCR reaction comprise NG primer, NT primer and the amplification primer,
wherein the NG primer and the NT primer comprise a universal sequence and a variable sequence from 5' end to 3' end, wherein the universal sequence consists of three or two of the four bases of G, A, C, and T, provided that the universal sequence does not comprise G and C at the same time;
the variable sequence of the NG primer is selected from a group consisting of: (N)nGGG, (N)xGTGG(N)y, or a combination thereof; and the variable sequence of the NT primer is selected from a group consisting of: (N)nTTT, (N) mTNTNG, or a combination thereof; wherein N is any nucleotide that can be base-paired with a natural nucleic acid, each n is independently a positive integer selected from 3-17, each m is independently a positive integer selected from 3-15, and each of x and y is a positive integer selected from 3-13, respectively;
whereas, the amplification primer contains the universal sequence without comprising the variable sequence.

## Patentansprüche

1. Verfahren zum Nachweisen der Chromosomenaberration in einem Embryo unter Verwendung von Blastozystenkulturflüssigkeit, das die folgenden Schritte umfasst:
(1) Erhalten einer Blastozystenkulturflüssigkeit: Kultivieren eines befruchteten Eis zum Blastomer-Stadium an Tag 3, und dann wird nach Überführen in ein neu hergestelltes Blastozystenkulturmikrotröpfchen für Blastozystenkulturen der die Blastozyste bildende Embryo entfernt und in eine neue Blastozystenkulturlösung oder ein vitrifiziertes Kryokonservierungsverfahren überführt, und die verbliebene Ausgangsblastozystenkulturflüssigkeit dient als Probe, die zum Nachweis entnommen wird;
(2) Entnehmen einer Blastozystenkulturflüssigkeit: Überführen der in Schritt (1) erhaltenen Ausgangsblastozystenkulturflüssigkeit in eine Lösung zur Lyse, und nach dem Zentrifugieren wird die Probe dem nächsten Schritt einer Gesamtgenomamplifikation ausgesetzt;
(3) Gesamtgenomamplifikation von Spuren-DNAs in Blastozystenkulturflüssigkeit: Lyase wird zu einer Mischung von der in Schritt (2) erhaltenen Blastozystenkulturflüssigkeit und einer Lösung zur Lyse gegeben, gemischt und inkubiert, die Lyase dann inaktiviert und das Lysat entfernt und in ein PCR-Reaktionsröhrchen für eine Genomamplifikationsreaktion gegeben;
(4) Analysieren durch Gesamtgenomamplifikation erhaltener DNA-Produkte, um zu bestimmen, ob der Chromosomenstatus des Embryos normal ist: Hochdurchsatzsequenzierung, Nukleinsäure-Chip oder Immunfluoreszenznachweis wird zur Analyse verwendet.

2. Verfahren nach Anspruch 1, wobei die Komponenten der Lösung zur Lyse in Schritt (2) 25-45 mM Tris-Cl mit einem pH von 7,0-8,0, 0,5-3 mM EDTA, 10-25 mM KCl und ein Detergens mit einer Konzentration von 0,05 %-5 % sind und es sich bei dem Detergens um eines oder mehrere, das/die aus einer Gruppe bestehend aus Triton X-100, Triton X-114, Tween 20, NP40 und SDS ausgewählt ist/sind, handelt.

3. Verfahren nach Anspruch 2, wobei die Komponenten der Lösung zur Lyse vorzugsweise 40 mM Tris-Cl, pH 7,2, 1 mM EDTA, 15 mM KCl und 3 % Triton X-100 sind.

4. Verfahren nach Anspruch 1, wobei es sich bei der Lyase in Schritt (3) um eine oder mehrere, die aus einer Gruppe bestehend aus Proteinase K, Qiagen Protease, Pepsin, Papain, Trypsin und Lysozym ausgewählt ist/sind, handelt und die Konzentration der Lyase 1-25 µg/ml beträgt.

5. Verfahren nach Anspruch 4, wobei die Konzentration der Lyase vorzugsweise 20 µg/ml beträgt.

6. Verfahren nach Anspruch 1, wobei die Inkubationstemperatur in Schritt (3) 30-60°C, die Inkubationszeit 1 Min. bis 12 Std., die Inaktivierungstemperatur 75-95°C und die Inaktivierungszeit 1-15 Min. beträgt.

7. Verfahren nach Anspruch 6, wobei vorzugsweise in Schritt (3) die Inkubationstemperatur 40°C, die Inkubationszeit 3 Std., die Inaktivierungstemperatur 90°C und die Inaktivierungszeit 5 Min. beträgt.

8. Verfahren nach Anspruch 1, wobei das PCR-Reaktionsröhrchen eine Amplifikationsmischung, 0,5 %-20 % eines PCR-Hemmerantagonisten, 5-20 mM dNTP, 5-100 µM NG- und NT-Primer, 50-200 µM Amplifikationsprimer, 0,5-10 Einheiten Nukleinsäurepolymerase umfasst, wenn die PCR-Reaktion in Schritt (3) durchgeführt wird, und es sich bei dem PCR-Hemmerantagonisten um einen oder mehrere, der/die aus einer Gruppe bestehend aus DMSO, Betain, Formamid, Glycerin und Albumin ausgewählt ist/sind, handelt, es sich bei der Nukleinsäurepolymerase um eine oder mehrere, die aus einer Gruppe bestehend aus Phi29-DNA-Polymerase, Bst-DNA-Polymerase, Vent-Polymerase, Deep-Vent-Polymerase, Klenow-Fragment-DNA-Polymerase I, MMLVreverse-Transkriptase, AMV-reverse-Transkriptase, HIV-reverse-Transkriptase, Phusion^{®} super-fidelity DNA-Polymerase, Taq-Polymerase, E.coli-DNA-Polymerase, LongAmp-Taq-DNA-Polymerase und OneTaq-DNA-Polymerase ausgewählt ist/sind, handelt.

9. Verfahren nach Anspruch 8, wobei die Komponenten der Amplifikationsmischung 10-25 mM Tris-HCl, 5-25 mM (NH4)2SO4, 5-30 mM KCl, 0,5-5 mM MgSO4, 0,1 %-20 % DMSO und 0,5-5 % Triton X-100 sind.

10. Verfahren nach Anspruch 9, wobei die Komponenten der Amplifikationsmischung vorzugsweise 15 mM Tris-HCl, 15 mM (NH4)2SO4, 20 mM KCl, 1 mM MgSO4, 5 % DMSO und 2 % Triton X-100 sind.

11. Verfahren nach Anspruch 8, wobei die NG- und NT-Primer eine universelle Sequenz und eine variable Sequenz vom 5'-Ende zum 3'-Ende umfassen und wobei die universelle Sequenz aus 3 oder 2 der 4 Basen G, A, C und T besteht, sofern die universelle Sequenz G und C nicht gleichzeitig umfasst; und der Ampflikationsprimer die universelle Sequenz, aber nicht die variable Sequenz umfasst.

12. Verfahren nach Anspruch 11, wobei die variable Sequenz aus einer Gruppe bestehend aus (N)nGGG, (N)nTTT, (N)mTNTNG, (N)xGTGG(N)y ausgewählt ist, wobei N für jegliche Nukleotide steht, die mit einer natürlichen Nukleinsäure eine Basenpaarung eingehen können, n für eine positive ganze Zahl, ausgewählt aus 3-17, steht, m für eine positive ganze Zahl, ausgewählt aus 3-15, steht und x und y jeweils für eine positive ganze Zahl, ausgewählt aus 3-13, stehen.

13. Verfahren nach Anspruch 12, wobei die NG- und NT-Primer die Sequenzen unter SEQ ID NO: 1 [GTGAGTGATGGTTGAGGTAGTGTGGAGNNNNNNNN], SEQ ID NO: 2 [GTGAGTGATGGTTGAGGTAGTGTGGAGNNNNNGGG], SEQ ID NO: 3 [GTGAGTGATGGTTGAGGTAGTGTGGAGNNNNNTTT], SEQ ID NO: 4 [GTGAGTGATGGTTGAGGTAGTGTGGAGNNNTNTNG] oder SEQ ID NO: 5 [GTGAGTGATGGTTGAGGTAGTGTGGAGNNNGTGGNN] umfassen, wobei N für jegliche Nukleotide steht, die mit einer natürlichen Nukleinsäure eine Basenpaarung eingehen können, und der Amplifikationsprimer die Sequenz unter SEQ ID NO: 6 [GTGAGTGATGGTTGAGGTAGTGTGGAG] von 5' nach 3' aufweist.

14. Verfahren nach Anspruch 1, wobei die Thermocycling-Technik der Gesamtgenomamplifikation in Schritt (3) wie folgt dargestellt ist:
(1) Umsetzen bei einer ersten Denaturierungstemperatur zwischen 90-98°C für 5-20 Sekunden;
(2) Umsetzen bei einer ersten Annealing-Temperatur von 5-15°C für 5-60 s, Umsetzen bei einer zweiten Annealing-Temperatur von 15-25°C für 5-60 s, Umsetzen bei einer dritten Annealing-Temperatur von 25-35°C für 30-80 s, Umsetzen bei einer vierten Annealing-Temperatur von 35-45°C für 5-60 s und Umsetzen bei einer fünften Annealing-Temperatur von 45-55°C für 5-60 s;
(3) Umsetzen bei einer ersten Verlängerungstemperatur von 55-80°C für 10-150 Min.;
(4) Umsetzen bei einer zweiten Denaturierungstemperatur von 90-98°C für 5-30 s;
(5) Umsetzen bei einer sechsten Annealing-Temperatur von 45-70°C für 10-30 s;
(6) Umsetzen bei einer zweiten Verlängerungstemperatur von 60-80°C für 1-10 Minuten;
(7) Wiederholen der Schritte (4) bis (6) über 5 bis 50 Zyklen;
(8) Fortsetzen der Verlängerungsreaktion bei einer Temperatur von 60-80°C für 1-10 Min.; und
(9) Kühlstellen und Lagern des amplifizierten Produkts bei 0-5°C.

15. Verfahren nach Anspruch 14, wobei die Thermocycling-Technik der Gesamtgenomamplifikation in Schritt (3) wie folgt dargestellt ist:
(1) Umsetzen bei einer ersten Denaturierungstemperatur zwischen 95°C für 10 Sekunden;
(2) Umsetzen bei einer ersten Annealing-Temperatur von 10°C für 45 s, Umsetzen bei einer zweiten Annealing-Temperatur von 20°C für 45 s, Umsetzen bei einer dritten Annealing-Temperatur von 30°C für 60 s, Umsetzen bei einer vierten Annealing-Temperatur von 40°C für 45 s und Umsetzen bei einer fünften Annealing-Temperatur von 50°C für 45 s;
(3) Umsetzen bei einer ersten Verlängerungstemperatur von 62°C für 90 Min.;
(4) Umsetzen bei einer zweiten Denaturierungstemperatur von 95°C für 20 s;
(5) Umsetzen bei einer sechsten Annealing-Temperatur von 59°C für 20 s;
(6) Umsetzen bei einer zweiten Verlängerungstemperatur von 72°C für 3 Min.;
(7) Wiederholen der Schritte (4) bis (6) über 10 bis 30 Zyklen;
(8) Fortsetzen der Verlängerungsreaktion bei einer Temperatur von 72°C für 5 Min.; und
(9) Kühlstellen und Lagern des amplifizierten Produkts bei 4°C.

16. Verfahren nach Anspruch 1, wobei, in Schritt (3), die in der PCR-Reaktion verwendeten Primer NG-Primer, NT-Primer und den Amplifikationsprimer umfassen,
wobei der NG-Primer und der NT-Primer eine universelle Sequenz und eine variable Sequenz vom 5'-Ende zum 3'-Ende umfassen, wobei die universelle Sequenz aus drei oder zwei der vier Basen G, A, C und T besteht, sofern die universelle Sequenz G und C nicht zur gleichen Zeit umfasst;
die variable Sequenz des NG-Primers aus einer Gruppe bestehend aus (N)nGGG, (N)xGTGG(N)y oder einer Kombination davon ausgewählt ist; und die variable Sequenz des NT-Primers aus der Gruppe bestehend aus (N)nTTT, (N)mTNTNG oder einer Kombination davon ausgewählt ist; wobei N für jegliche Nukleotide steht, die mit einer natürlichen Nukleinsäure eine Basenpaarung eingehen können, n jeweils unabhängig für eine positive ganze Zahl, ausgewählt aus 3-17, steht, m jeweils unabhängig für eine positive ganze Zahl, ausgewählt aus 3-15, steht und x und y jeweils für eine positive ganze Zahl, ausgewählt aus 3-13, stehen;
wohingegen der Amplifikationsprimer die universelle Sequenz enthält, ohne die variable Sequenz zu umfassen.

## Revendications

1. Procédé de détection de l'anomalie chromosomique dans un embryon en utilisant un fluide de culture de blastocystes, comprenant les étapes de :
(1) obtention d'un fluide de culture de blastocystes : culture d'un œuf fécondé au stade blastomère le jour 3, puis transféré dans une microgouttelette de culture de blastocystes nouvellement préparée pour la culture de blastocystes, l'embryon qui forme le blastocyste étant retiré et transféré dans une nouvelle solution de culture de blastocystes ou dans un processus de cryoconservation vitrifiée, et le fluide de culture de blastocystes original restant étant un échantillon à collecter pour la détection ;
(2) collecte d'un fluide de culture de blastocystes : transfert du fluide de culture de blastocystes original obtenu à l'étape (1) dans une solution de lyse, et après centrifugation, l'échantillon est soumis à l'étape suivante d'amplification du génome entier ;
(3) amplification du génome entier d'ADN de traces dans le fluide de culture de blastocystes : une lyase est ajoutée à un mélange du fluide de culture de blastocystes obtenu à l'étape (2) et d'une solution de lyse, mélangée et incubée, puis la lyase est inactivée et le lysat est retiré et ajouté à un tube de réaction de PCR pour une réaction d'amplification du génome ;
(4) analyse de produits d'ADN obtenus de l'amplification du génome entier pour déterminer si le statut chromosomique de l'embryon est normal: séquençage de deuxième génération, puce d'acide nucléique ou détection par immunofluorescence sont utilisés pour l'analyse.

2. Procédé selon la revendication 1, dans lequel les composants de la solution de lyse dans l'étape (2) sont 25-45 mM de Tris-Cl ayant un pH de 7,0 à 8,0, 0,5-3 mM d'EDTA, 10-25 mM de KCl et un détergent ayant une concentration de 0,05 % à 5 %, et le détergent est un ou plusieurs choisis dans le groupe constitué par Triton X-100, Triton X-114, Tween 20, NP40, et SDS.

3. Procédé selon la revendication 2, dans lequel les composants de la solution de lyse sont de préférence 40 mM de Tris-Cl, pH 7,2, 1 mM d'EDTA, 15 mM de KCl, et 3 % de Triton X-100.

4. Procédé selon la revendication 1, dans lequel la lyase dans l'étape (3) est une ou plusieurs choisies dans le groupe constitué par la protéinase K, la protéase Oiagen, la pepsine, la papaïne, la trypsine et le lysozyme, et la concentration de la lyase est de 1-25 µg/ml.

5. Procédé selon la revendication 4, dans lequel la concentration de la lyase est de préférence de 20 µg/ml.

6. Procédé selon la revendication 1, dans lequel la température d'incubation dans l'étape (3) est de 30-60 °C, la durée d'incubation est de 1 min à 12 h, la température d'inactivation est de 75-95 °C, et la durée d'inactivation est de 1-15 min.

7. Procédé selon la revendication 6, dans lequel de préférence, dans l'étape (3), la température d'incubation est de 40 °C, la durée d'incubation est de 3 heures, la température d'inactivation est de 90 °C, et la durée d'inactivation est de 5 min.

8. Procédé selon la revendication 1, dans lequel, lorsque la réaction de PCR est effectuée dans l'étape (3), le tube de réaction de PCR comprend un mélange d'amplification, 0,5 % à 20 % d'un antagoniste inhibiteur de PCR, 5-20 mM de dNTP, 5-100 µM d'amorces NG et NT, 50-200 µM d'amorces d'amplification, 0,5-10 unités d'acide nucléique polymérase, et l'antagoniste inhibiteur de PCR est un ou plusieurs choisis dans un groupe constitué par le DMSO, la bétaïne, le formamide, le glycérol et l'albumine, la polymérase d'acide nucléique est une ou plusieurs choisies dans un groupe constitué par l'ADN polymérase Phi29, l'ADN polymérase Bst, la polymérase Vent, la polymérase Deep Vent, l'ADN polymérase | du fragment Klenow, la transcriptase inverse MMLV, la transcriptase inverse AMV, la transcriptase inverse VIH, l'ADN polymérase super-fidélité Phusion^{®}, la polymérase Taq, l'ADN polymérase E.coli, l'ADN polymérase LongAmp Taq, et l'ADN polymérase OneTaq.

9. Procédé selon la revendication 8, dans lequel les composants du mélange d'amplification sont 10-25 mM de Tris-HCl, 5-25 mM de (NH4)2SO4, 5-30 mM de KCl, 0,5-5 mM de MgSO₄, 0,1 % à 20 % de DMSO et 0,05 % à 5 % de Triton X-100.

10. Procédé selon la revendication 9, dans lequel les composants du mélange d'amplification sont de préférence 15 mM de Tris-HCl, 15 mM de (NH4)2SO4, 20 mM de KCl, 1 mM de MgSO₄, 5 % de DMSO et 2 % de Triton X-100.

11. Procédé selon la revendication 8, dans lequel les amorces NG et NT comprennent une séquence universelle et une séquence variable de l'extrémité 5' à l'extrémité 3', et dans lequel la séquence universelle est constituée de 3 ou 2 des 4 bases parmi G, A, C et T, à condition que la séquence universelle ne comprenne pas simultanément G et C; et l'amorce d'amplification comprend la séquence universelle mais ne comprend pas la séquence variable.

12. Procédé selon la revendication 11, dans lequel la séquence variable est choisie dans un groupe constitué par: (N)nGGG, (N)nTTT, (N)mTNTNG, (N)xGTGG(N)y, dans lequel N est un quelconque nucléotide qui peut être apparié en bases avec un acide nucléique naturel, n est un entier positif choisi parmi 3 à 17, m est un entier positif choisi parmi 3 à 15, et chacun de x et y est un entier positif choisi parmi 3 à 13, respectivement.

13. Procédé selon la revendication 12, dans lequel les amorces NG et NT comprennent la séquence de SEQ ID NO: 1 [GTGAGTGATGGTTGAGGTAGTGTGGAGNNNNNNNN], SEQ ID NO: 2 [GTGAGTGATGGTTGAGGTAGTGTGGAGNNNNNGGG], SEQ ID NO: 3 [GTGAGTGATGGTTGAGGTAGTGTGGAGNNNNNTTT], SEQ ID NO: 4 [GTGAGTGATGGTTGAGGTAGTGTGGAGNNNTNTNG], ou SEQ ID NO: 5 [GTGAGTGATGGTTGAGGTAGTGTGGAGNNNGTGGNN], dans lesquelles N est un quelconque nucléotide qui peut être apparié en bases avec un acide nucléique naturel; et l'amorce d'amplification a la séquence SEQ ID NO: 6 [GTGAGTGATGGTTGAGGTAGTGTGGAG] de 5' à 3'.

14. Procédé selon la revendication 1, dans lequel la procédure de thermocyclage de l'amplification du génome entier dans l'étape (3) est présentée comme suit :
(1) mise en réaction à une première température de dénaturation comprise entre 90-98 °C pendant 5-20 secondes ;
(2) mise en réaction à une première température d'annelage de 5-15 °C pendant 5-60 s, mise en réaction à une deuxième température d'annelage de 15-25 °C pendant 5-60 s, mise en réaction à une troisième température d'annelage de 25-35 °C pendant 30-80 s, mise en réaction à une quatrième température d'annelage de 35-45 °C pendant 5-60 s, et mise en réaction à une cinquième température d'annelage de 45-55 °C pendant 5-60 s;
(3) mise en réaction à une première température d'extension de 55-80 °C pendant 10-150 min;
(4) mise en réaction à une deuxième température de dénaturation de 90-98 °C pendant 5-30 s;
(5) mise en réaction à une sixième température d'annelage de 45-70 °C pendant 10-30 s;
(6) mise en réaction à une deuxième température d'extension de 60-80 °C pendant 1-10 minutes;
(7) répétition des étapes (4) à (6) pendant 5 à 50 cycles;
(8) continuation de la réaction d'extension à une température de 60-80 °C pendant 1-10 min; et
(9) réfrigération et stockage du produit amplifié à 0-5 °C.

15. Procédé selon la revendication 14, dans lequel la procédure de thermocyclage de l'amplification du génome entier dans l'étape (3) est présentée comme suit :
(1) mise en réaction à une première température de dénaturation comprise entre 95 °C pendant 10 secondes ;
(2) mise en réaction à une première température d'annelage de 10 °C pendant 45s, mise en réaction à une deuxième température d'annelage de 20 °C pendant 45 s, mise en réaction à une troisième température d'annelage de 30 °C pendant 60 s, mise en réaction à une quatrième température d'annelage de 40 °C pendant 45 s, et mise en réaction à une cinquième température d'annelage de 50 °C pendant 45 s;
(3) mise en réaction à une première température d'extension de 62 °C pendant 90 min;
(4) mise en réaction à une deuxième température de dénaturation de 95 °C pendant 20 s;
(5) mise en réaction à une sixième température d'annelage de 59 °C pendant 20 s;
(6) mise en réaction à une deuxième température d'extension de 72 °C pendant 3 min;
(7) répétition des étapes (4) à (6) pendant 10 à 30 cycles;
(8) continuation de la réaction d'extension à une température de 72 °C pendant 5 min; et
(9) réfrigération et stockage du produit amplifié à 4 °C.

16. Procédé selon la revendication 1, dans lequel, à l'étape (3), les amorces utilisées dans la réaction de PCR comprennent une amorce NG, une amorce NT et l'amorce d'amplification,
dans lequel l'amorce NG et l'amorce NT comprennent une séquence universelle et une séquence variable de l'extrémité 5' à l'extrémité 3', dans lequel la séquence universelle est constituée de trois ou deux des quatre bases parmi G, A, C et T, à condition que la séquence universelle ne comprenne pas simultanément G et C ;
la séquence variable de l'amorce NG est sélectionnée dans un groupe constitué par: (N)nGGG, (N)xGTGG(N)y, ou une combinaison correspondante; et la séquence variable de l'amorce NT est choisie dans un groupe constitué par : (N)nTTT, (N) mTNTNG, ou une combinaison correspondante ; dans lequel N est un quelconque nucléotide qui peut être apparié en bases avec un acide nucléique naturel, chaque n est indépendamment un entier positif choisi parmi 3 à 17, chaque m est indépendamment un entier positif choisi parmi 3 à 15, et chacun de x et y est un entier positif choisi parmi 3 à 13, respectivement ;
tandis que l'amorce d'amplification contient la séquence universelle sans comprendre la séquence variable.
